# EUROPEAN PATENT SPECIFICATION

(11) **EP 4 125 693 B1**
(45) Date of publication and mention of the grant of the patent: **22.07.2026**
(21) Application number: 21716837.6
(22) Date of filing: 29.03.2021
(51) Int. Cl.: A61B 34/37, B25J 9/16, A61B 34/00, B25J 13/08

(54) **CONFIGURING A SURGICAL ROBOTIC SYSTEM**
KONFIGURATION EINES CHIRURGISCHEN ROBOTERSYSTEMS
CONFIGURATION D'UN SYSTÈME ROBOTIQUE CHIRURGICAL

(30) Priority: 31.03.2020 GB 202004754
(43) Date of publication of application: 08.02.2023
(73) Proprietor: CMR Surgical Limited, Cambridge CB24 9NG (GB)
(72) Inventor: SCHOLAN, Andrew Murray, Cambridge Cambridgeshire CB24 9NG (GB); HARES, Luke David Ronald, Cambridge Cambridgeshire CB24 9NG (GB)
(74) Representative: Slingsby Partners LLP
(86) International application number: PCT/GB2021/050767
(87) International publication number: WO 2021/198663

(56) References cited:
- WO-A1-2014/020571
- WO-A1-2016/116753
- WO-A1-2019/050821
- CN-B- 105 616 007
- JP-B1- 6 469 304
- US-A1- 2013 116 706
- US-A1- 2019 202 066

## Description

### BACKGROUND

This invention relates to a control system for reconfiguring a surgical robotic system.

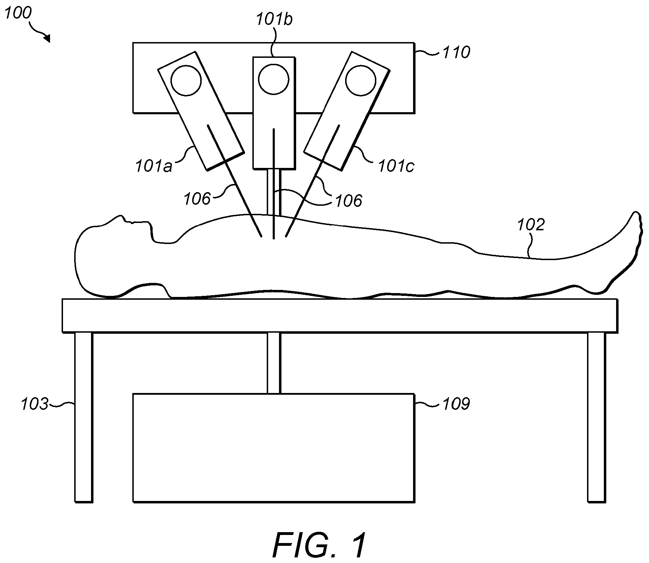

Invasive medical procedures can be performed using surgical robotic systems. Figure 1 shows a typical surgical robotic system. The surgical robotic system 100 is shown performing an invasive medical procedure on a patient 102 positioned on an operating table 103. The surgical robotic system 100 comprises three arms 101a, 101b and 101c. The three arms 101a, 101b, 101c attach to a common unit 110. Each arm 101a, 101b and 101c may carry a surgical tool 106, such as a tool for performing cutting or grasping or an imaging device such as an endoscope. Each arm 101a, 101b, 101c may manipulate the surgical tool that it carries in order to perform aspects of the invasive procedure. The surgical robotic system is supported by a base 109 resting on the floor of the operating room.

In the event that one or more of arms 101a, 101b or 101c develop a fault, or are no longer useable for any other reason, often a decision must be made as to whether: (i) the invasive procedure can be completed using only the remaining arms of the surgical robotic system; or (ii) the invasive procedure should be converted to a manual procedure (e.g. in which a surgeon, rather than the robotic surgical system, manipulates surgical tools in order to complete the procedure). Both of these scenarios are undesirable - both lead to an increased difficulty in completing the invasive procedure, and converting to a manual procedure often leads to a longer recovery time for the patient.

Thus, it would be desirable if there were an improved method of reconfiguring a surgical robotic system such that the abovementioned problems can be addressed. JP 6469304 relates to a surgery assistance device, a control method thereof, and a program.

WO 2016/116753 relates to a robot and control of that robot's movement of an attached tool.

WO 2019/050821 relates to surgical robotic systems, methods, and computer-readable media for robotic surgery and improving user experience while performing surgeries using surgical robotic systems.

CN 105616007 relates generally to medical robotic systems and, in particular, to medical robotic systems that provide linked control modes.

US 2013/116706 relates to a surgical robot used for minimally invasive surgery and a method for controlling the same.

### SUMMARY OF THE INVENTION

According to the invention there is provided a control system of a surgical robotic system, the surgical robotic system comprising a first robot arm and a second robot arm, each of the first and second robot arms comprising a series of joints by which the configuration of that robot arm can be altered, the series of joints extending from a base at a proximal end of the robot arm to an attachment for a surgical instrument at a distal end of the robot arm, the control system being configured to reconfigure the surgical robotic system by: controlling the first robot arm to operate in a surgical mode in which a first surgical instrument attached to that first robot arm is inside a patient's body; and whilst the first robot arm is operating in the surgical mode: (i) controlling the second robot arm so as to permit a second surgical instrument attached to the second robot arm to be inserted into a port in the patient's body; (ii) determining a fulcrum about which the second surgical instrument pivots when the configuration of the second robot arm is altered whilst the second surgical instrument is inside the port; and (iii) controlling the second robot arm to operate in a surgical mode in which the configuration of the second robot arm and second surgical instrument is controlled in response to inputs received at a remote surgeon console whilst maintaining an intersection between the second surgical instrument and the determined fulcrum.

The fulcrum may be determined by: controlling the second robot arm so as to enable its configuration to be altered in response to external forces whilst the second surgical instrument is inside the port; and determining the fulcrum, the fulcrum being the point about which the surgical instrument of the second robot arm pivots whilst inside the port.

The second robot arm may further comprise one or more force sensors configured to sense external forces at one or more joints of the series of joints of the second robot arm, and one or more motors configured to drive one or more joints of the series of joints of the second robot arm, and the control system may be further configured to: control the one or more motors so as to drive one or more joints of the series of joints of the second robot arm in dependence on external forces sensed by the one or more force sensors so as to alter the configuration of the second robot arm.

The second robot arm may further comprise one or more position sensors configured to sense the position of one or more joints of the series of joints of the second robot arm and to record the position of one or more joints of the series of joints of the second robot arm at a plurality of instances whilst the configuration of the second robot arm is being altered, and the control system further may be configured to: determine, for each instance, a position of the distal end of the second robot arm in dependence on the respective recorded one or more joint positions; determine, for each instance, a vector of the second surgical instrument from the determined position of the distal end of the second robot arm in dependence on the respective recorded one or more joint positions; and determine the point of intersection of the determined vectors of the second surgical instrument so as to determine the fulcrum.

The control system may be further configured to: determine the fulcrum when controlling the second robot arm to operate in a calibration mode; and control the second robot arm to transition from operating in the calibration mode to operating in the surgical mode.

The second robot arm may further comprise a more distal interface and a less distal interface, and the control system may be further configured to: control the second robot arm to transition from operating in the calibration mode to operating in the surgical mode in response to an operator interaction with the more distal interface.

The control system may be further configured to: control the second robot arm so as to permit the second surgical instrument to be inserted into the port by controlling the second robot arm to operate in a compliant mode in which the configuration of the second robot arm can be altered in response to external forces; and control the second robot arm to transition from operating in the compliant mode to operating in the calibration mode.

The control system may be further configured to: control the second robot arm to transition from operating in the compliant mode to operating in the calibration mode in response to a user interaction with the more distal interface.

The control system may be further configured to: after determining the fulcrum, control the second robot arm to operate in an instrument adjust mode in which the configuration of the second robot arm can be altered in response to external forces but is constrained such that an intersection is maintained between the second surgical instrument and the determined fulcrum.

The control system may be further configured to: control the second robot arm to transition from operating in the calibration mode to operating in the instrument adjust mode; and control the second robot arm to transition from operating in the instrument adjust mode to operating in the surgical mode, and optionally control the second robot arm to transition from operating in the surgical mode to operating in the instrument adjust mode.

Each of the first robot arm and second robot arm may further comprise an orientation interface, and the control system may be further configured to: receive an input identifying a common direction in response to an operator indicating a direction using the orientation interface of the first robot arm and indicating a corresponding direction using the orientation interface of the second robot arm.

In the surgical mode, the second robot arm may be remotely controlled by the control system being configured to: receive inputs relating to the second robot arm to the remote console; convert the inputs into control signals for the second robot arm in dependence on the determined fulcrum and the identified common direction; and control one or more joints of the series of joints of the second robot arm in dependence on the control signals so as to control the configuration of the second robot arm.

The surgical robotic system may comprise a third robot arm comprising a series of joints by which the configuration of that robot arm can be altered, the series of joints extending from a base at a proximal end of the robot arm to an attachment for a surgical instrument at a distal end of the robot arm, and the control system may be further configured to: whilst controlling the first robot arm to operate in the surgical mode and prior to permitting the second surgical instrument to be inserted into the port, control the third robot arm so as to permit a third surgical instrument attached to the third robot arm to be retracted from the patient's body.

The control system may be further configured to permit the third surgical instrument to be retracted from the patient's body by: enabling the configuration of the third robot arm to be altered in response to external forces, the freedom of motion of the third robot arm being limited such that the third surgical instrument can only move linearly in directions co-axial with the longitudinal axis of the third surgical instrument and away from the patient's body.

The third robot arm may further comprise one or more force sensors configured to sense external forces at one or more joints of the series of joints of the third robot arm, and one or more motors configured to drive one or more joints of the series of joints of the third robot arm, and the control system may be further configured to: resolve external forces sensed by the one or more force sensors so as to determine the components of the forces parallel with the longitudinal axis of the third surgical instrument and away from the patient's body; and control the one or more motors so as to drive one or more joints of the series of joints of the third robot arm in dependence on the components of the forces parallel with the longitudinal axis of the third surgical instrument so as to alter the configuration of the third robot arm.

The control system may be further configured to, whilst controlling the first robot arm to operate in the surgical mode and prior to permitting the second surgical instrument to be inserted into the port, control the second robot arm so as to permit the second surgical instrument to be retracted from the patient's body; and control the second robot arm so as to permit the second surgical instrument to be inserted into the patient's body after a maintenance task has been performed on the second robot arm.

The control system may be further configured to permit the second surgical instrument to be retracted from the patient's body by: enabling the configuration of the second robot arm to be altered in response to external forces, the freedom of motion of the second robot arm being limited such that the second surgical instrument can only move linearly in directions parallel with the longitudinal axis of the second surgical instrument.

The second robot arm may further comprise one or more force sensors configured to sense external forces at one or more joints of the series of joints of the second robot arm, and one or more motors configured to drive one or more joints of the series of joints of the second robot arm, and the control system may be further configured to: resolve external forces sensed by the one or more force sensors so as to determine the components of the forces parallel with the longitudinal axis of the second surgical instrument; and control the one or more motors so as to drive one or more joints of the series of joints of the second robot arm in dependence on the components of the forces parallel with the longitudinal axis of the second surgical instrument so as to alter the configuration of the second robot arm.

The surgical mode in which the control system controls the first robot arm to operate is an engaged surgical mode in which the configuration of the first robot arm and first surgical instrument is controlled in response to inputs received at the remote surgeon console. In an alternative embodiment, not part of the claimed subject-matter, the surgical mode is a disengaged surgical mode in which the configuration of the first robot arm and first surgical instrument is controllable in response to inputs received at the remote surgeon console.

### BRIEF DESCRIPTION OF THE DRAWINGS

The present invention will now be described by way of example with reference to the accompanying drawings. In the drawings:
Figure 1 shows a typical surgical robotic system.
Figure 2 shows a surgical robotic system.
Figure 3 shows a surgical robot arm of a surgical robotic system.
Figure 4 shows a start-up sequence of modes for a surgical robot arm.
Figure 5 is a flow diagram showing the steps for calibrating a surgical robot arm.
Figure 6 shows a plan view of a surgical robotic system.
Figure 7 shows a flow diagram for reconfiguring a surgical robotic system in accordance with the principles described herein.

### DETAILED DESCRIPTION OF THE DRAWINGS

The following description is presented to enable any person skilled in the art to make and use the invention, and is provided in the context of a particular application. Various modifications to the disclosed embodiments will be readily apparent to those skilled in the art.

The general principles defined herein may be applied to other embodiments and applications without departing from the scope of the present invention. Thus the present invention is not intended to be limited to the embodiments shown, but is to be accorded the widest scope consistent with the principles and features disclosed herein.

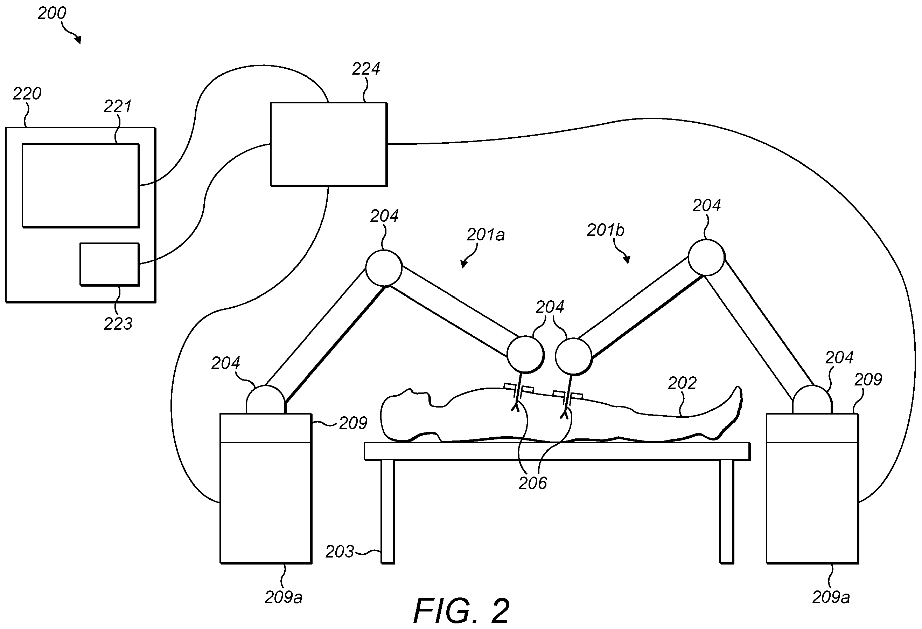

Figure 2 shows a surgical robotic system. Figure 2 shows a surgical robotic system 200 performing an invasive medical procedure on a patient 202. The patient 202 is positioned on an operating table 203. The surgical robotic system 100 comprises a first robot arm 201a and a second robot arm 201b. Although two robot arms 201a, 201b are shown in Figure 2, it is to be understood that a surgical robotic system configured in accordance with the principles described herein may comprise any number of robot arms. Each robot arm 201a, 201b extends from a base 209 at its proximal end. Each robot arm 201a, 201 b comprises a plurality of joints 204 by which the configuration of that robot arm can be altered.

Each robot arm 201a, 201b comprises an attachment for a surgical instrument 206 at its distal end. The surgical instrument may have a thin elongate shaft with an end effector at its distal end for performing aspects of the invasive procedure. The surgical instrument could, for example, be a cutting or grasping device, or an imaging device (such as an endoscope). Each surgical instrument 206 is insertable into a patient's body 202. Each surgical instrument 206 may be inserted into a patient's body 202 via a port. Each surgical instrument 206 may be inserted into the patient 202 through a different port.

The base 209 of each robot arm 201a, 201b is supported by a support structure 209a. The support structure 209a may be moveable. The support structure may be a cart or trolley. For example, each support structure may comprise one or more wheels (not shown) on which the support structure can be moved. In other examples, a moveable support structure may be mounted on one or more of: rails, sliders or bearings, or any other element on which the support structure can be moved. In an unshown example, the base 209a may be supported by a wall or ceiling mounted arm support structure (e.g. by being secured to that structure). Said wall or ceiling mounted arm support structure may be mounted on one or more rails, or any other suitable element, such that the wall or ceiling mounted arm support structure can be moved. An arm support structure may be moveably mounted on any other suitable surface (e.g. operating table 203). The moveable support structure may be equipped with brakes (not shown), such that the support structure can be caused to remain static when required (e.g. whilst the robot arm it supports is participating in an invasive medical procedure).

The configuration of each robot arm 201a, 201b may be remotely controlled in response to inputs received at a remote surgeon console 220. A surgeon may provide inputs to the remote console 220. The remote surgeon console comprises one or more surgeon input devices 223. For example, these may take the form of a hand controller and/or foot pedal. The surgeon console also comprises a display 221.

A control system 224 connects the surgeon console 220 to each surgical robot 201a, 201b. The control system receives inputs from the surgeon input device(s) and converts these to control signals to move the joints of the robot arms 104 and surgical instrument 206. The control system 224 sends these control signals to the robot, where the corresponding joints are driven accordingly. The control system 224 may be separate from the remote surgeon console 220 and the robot arms 201a, 201b. The control system 224 may be collocated with the remote surgeon console 220. The control system 224 may be collocated with one of the robot arms 201a, 201b. The control system 224 may be distributed between the remote surgeon console 220 and the robot arms 201a, 201b.

The configuration of each robot arm 201a, 201b may be controllable in response to external forces applied directly to that robot arm. For example, a member of the bedside team (e.g. an operating room nurse) may apply forces directly to a robot arm (e.g. by pushing a joint of the robot arm). This behaviour will be described in further detail herein.

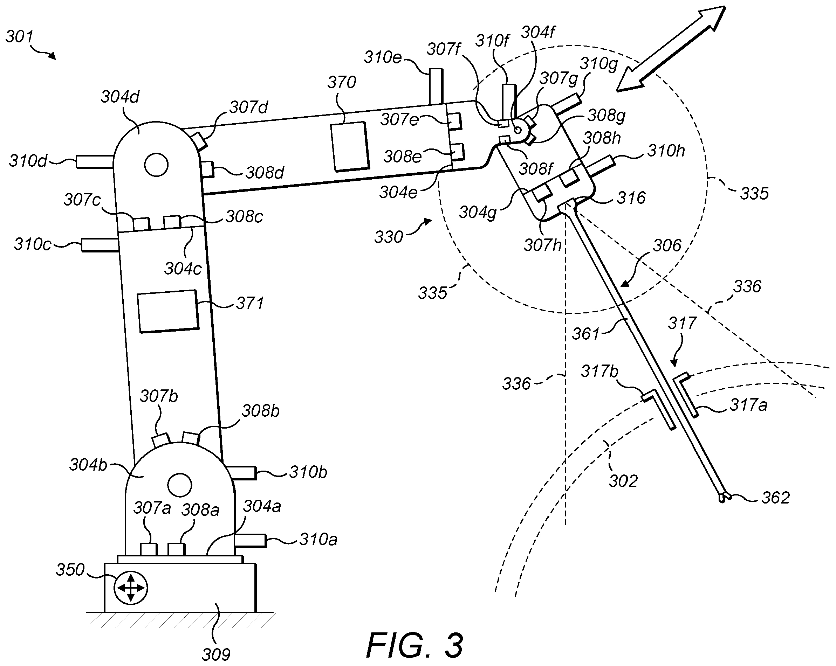

Figure 3 shows an example of a robot arm 301. The robot arms 201a, 201b shown in Figure 2 may have the same features as the robot arm 301 shown Figure 3.

The robot arm comprises a base 309. The robot arm has a series of rigid arm members. Each arm member in the series is joined to the preceding arm member by a respective joint 304a-g. Joints 304a-e and 304g are revolute joints. Joint 304f is composed of two revolute joints whose axes are orthogonal to each other, as in a Hooke's or universal joint. Joint 304f may be termed a "wrist joint". A robot arm could be jointed differently from the arm of Figure 3. For example, joint 304d could be omitted and/or joint 304f could permit rotation about a single axis. The robot arm could include one or more joints that permit motion other than rotation between respective sides of the joint, such as a prismatic joint by which an instrument attachment can slide linearly with respect to more proximal parts of the robot arm.

The joints are configured such that the configuration of the robot arm can be altered allowing the distal end 330 of the robot arm to be moved to an arbitrary point in a three-dimensional working volume illustrated generally at 335. One way to achieve that is for the joints to have the arrangement illustrated in Figure 3. Other combinations and configurations of joints could achieve a similar range of motion, at least within the zone 335. There could be more or fewer arm members.

The distal end of the robot arm 330 has an attachment 316 by means of which a surgical instrument 306 can be releasably attached. The surgical instrument has a linear rigid shaft 361 and an end effector 362 at the distal end of the shaft. The end effector 362 consists of a device for engaging in a procedure, for example a cutting, grasping or imaging device. As described herein, terminal joint 304g may be a revolute joint. The surgical instrument 306 and/or the attachment 316 may be configured so that the instrument extends linearly parallel with the rotation axis of the terminal joint 304g of the robot arm. In this example the instrument extends along an axis coincident with the rotation axis of joint 304g.

Joints 304e and 304f of the robot arm are configured so that with the distal end of the robot arm 330 held at an arbitrary location in the working volume 335 the surgical instrument 306 can be directed in an arbitrary direction within a cone. Such a cone is illustrated generally at 336. One way to achieve that is for the terminal part of the arm to comprise the pair of joints 304e and 304f whose axes are mutually arranged as described above. Other mechanisms can achieve a similar result. For example, joint 304g could influence the attitude of the instrument if the instrument extends In a direction which is not parallel to the axis of joint 304g.

The surgical instrument 306 may be inserted into the patient's body through a port 317. The port 317 may comprise a hollow tube 317a. The hollow tube 317a may pass through the outer tissues 302 of the patient so as to limit disruption to those tissues as the surgical instrument is inserted and removed, and as the instrument is manipulated within the patient's body. The port 317 may comprise a collar 317b. The collar 317b may prevent the port 317 being inserted too far through the outer tissues 302 of the patient. In other examples (not shown), the surgical instrument may be inserted directly into the patient's body, e.g. through a natural orifice such as the throat. A natural orifice through which the surgical instrument is inserted into the patient's body is also referred to herein as a "port".

The robot arm 301 comprises a series of motors 310a-h. With the exception of the compound joint 304f, which is served by two motors, each motor is arranged to drive rotation about a respective joint of the robot arm. The motors are controlled by a control system (such as control system 224 shown in Figure 2). The control unit comprises a processor and a memory. The memory stores, in a non-transient way, software code that can be executed by the processor to cause the processor to control the motors 310a-h in the manner described herein.

The robot arm 301 may comprise a series of sensors 307a-h and 308a-h. These sensors may comprise, for each joint, a position sensor 307a-h for sensing the rotational position of the joint and a force sensor 308a-h for sensing forces (such as torque) applied about the joint's rotation axis. Compound joint 304f may have two pairs of sensors. One or both of the position and force sensors for a joint may be integrated with the motor for that joint. The outputs of the sensors are passed to the control system where they form inputs for the processor.

The robot arm 301 may also comprise an orientation interface 350. The orientation interface 350 may be, for example, a button or set of buttons accessible by a member of the bedside team (e.g. an operating room nurse). Orientation interface 350 can be used to identify a common direction for the robot arms of a surgical robotic system (such as surgical robotic system 200 shown in Figure 2). The output of the orientation sensor may be passed to the control system where it forms an input to the processor. The orientation interface 350 will be described in further detail below.

The robot arm 301 may also comprise one or more interfaces 370, 371. Interfaces 370, 371 may be, for example, a button or set of buttons accessible by a member of the bedside team (e.g. an operating room nurse). Interfaces 370, 371 can be used to select between the operating modes of a robot arm. Interfaces 370, 371 will be described in further detail below.

### Configuring a robot arm

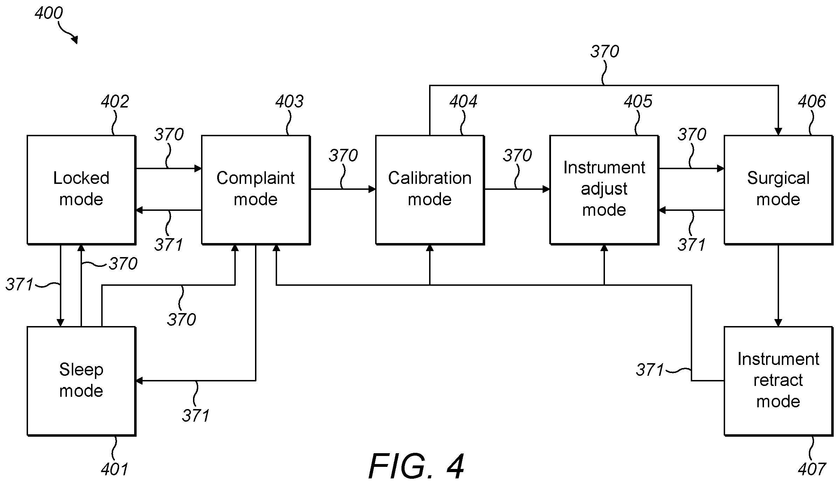

In order to configure a robot arm such that it can be used as part of a surgical robotic system, a start-up sequence of modes may be used. Figure 4 shows a start-up sequence of modes for a surgical robot arm. It is to be understood that the control system of the surgical robotic system (such as control system 224 shown in Figure 2) controls the operation of each robot arm in the surgical robotic system - i.e. in accordance with the various operating modes described with reference to Figure 4. It is to be understood that the control system controls the operation and behaviour of each robot arm in each operating mode, and controls the transitioning of each robot arm between operating modes as described herein.

As shown in Figure 4, a robot arm may be operated in a sleep mode 401, followed by a locked mode 402, followed by a compliant mode 403, followed by a calibration mode 404, followed by an adjust mode 405, followed by a surgical mode 406. That said, it is not necessary for a robot arm to be operated in all of the modes shown in Figure 4 in order to be configured such that it can be operated in a surgical mode 406. For example, a robot arm may be operated in the calibration mode 404, directly followed by the surgical mode 406. A robot arm may be sequentially operated in any combination of the modes shown in Figure 4, by performing any sequence mode transitions shown to be possible by the arrows in Figure 4. For example, a robot arm may be operated in the calibration mode 404, directly followed by the surgical mode 406, followed by the instrument adjust mode 405, followed by returning to the surgical mode 406.

The robot arm may be operable in a sleep mode 401. In the sleep mode 401, the robot arm may adopt a configuration suitable for storing or transporting the robot arm. Such a configuration may be a compact configuration. For example, in said configuration the proximal arm members may be substantially parallel to one another - although other compact configurations are also suitable. A surgical instrument 306 may not be attached to the robot arm when it is operating in the sleep mode 401.

In the sleep mode 401 the robot arm may resist external forces so as to maintain its configuration. Brakes may be applied at one or more of the joints 304a-g of the robot arm such that the robot arm can resist external forces so as to maintain its configuration. Said brakes may be of any suitable type, such as electronic, magnetic or mechanical brakes. In the sleep mode 401, the robot arm does not change its configuration in response to inputs at the remote surgeon console (e.g. remote surgeon console 220 shown in Figure 2).

The robot arm may be operable in a locked mode 402. In the locked mode, the robot arm may adopt a "horse-shoe" configuration (e.g. of the type shown in Figures 2 and 3). The "horse-shoe" configuration is preferable in the locked mode 402 as the orientation of the joints of the robot arm are away from their joint limits and singular configurations. That is, the "horse-shoe" configuration is such that a large range of movement of the robot arm is possible from that configuration. In the "horse-shoe" the robot arm can be placed near the port in a configuration which is similar to the final/optimal configuration that will be used when performing surgery - so that the operating staff have a good idea on how the surgical setup will look like. On transitioning from the sleep mode 401 to the locked mode 402, the configuration of the robot arm may be altered from a compact configuration to the "horse-shoe" configuration. Such a change may be driven by one or more of the series of motors 310a-h.

A surgical instrument 306 may be attached to the robot arm when it is operating in the locked mode 402. In the locked mode 402 the robot arm may resist external forces so as to maintain its configuration. Brakes may be applied at one or more of the joints 304a-g of the robot arm such that the robot arm can resist external forces so as to maintain its configuration. Said brakes may be of any suitable type, such as electronic, magnetic or mechanical brakes. In the locked mode 402, the robot arm does not change its configuration in response to inputs at the remote surgeon console (e.g. remote surgeon console 220 shown in Figure 2).

A surgical instrument 306 may be attached to the robot arm when it is operating in the locked mode 402. A drape (not shown) may be applied to the robot arm when it is operating in the locked mode 402.

The operating mode of the robot arm may also be transitioned from the locked mode 402 to the sleep mode 401, such as when the robot arm is being prepared for storage after a procedure has been completed.

The robot arm may be operable in a compliant mode 403. As shown in Figure 4, the operating mode of the robot arm may be caused to transition from the locked mode 402 to the compliant mode 403. The operating mode of the robot arm may also be caused to transition directly from the sleep mode 401 to the compliant mode 403. On transitioning from the sleep mode 401 to the compliant mode 403, the configuration of the robot arm may be changed from a compact configuration to the "horse-shoe" configuration.

In the compliant mode 403, the configuration of the robot arm is changeable in response to external forces applied directly to that robot arm. For example, a member of the bedside team (e.g. an operating room nurse) may apply forces directly to a robot arm (e.g. by pushing a joint of the robot arm). In the compliant mode 403 the control system (such as control system 224 shown in Figure 2) controls the robot arm to maintain a position in which it is placed by means of external forces applied directly to the robot arm.

To achieve this, the control system receives inputs from the position and force sensors 307a-h and 308a-h. From the position sensors the control system can determine the current configuration of the robot arm. The control system stores for each element of the robot arm, and the surgical instrument, its mass, the distance of its centre of mass from the preceding joint of the robot arm and the relationship between the centre of mass and the positional output of the position sensor for the preceding joint. The current configuration of the robot arm could be inferred by other means. For example, camera-based positioning systems may be used to track points in space, such as fiducial markers attached to the robot arm. This technique could be used to determine the joint angles. Other techniques include inferring the position of a joint using a current sensors. For example, the position of a joint can be inferred from the amount of current passing through the motor and assuming a given relationship to be constant.

Using that information, the control system models the effect of gravity on the components of the robot arm for the current configuration of the robot arm and estimates a force (e.g. a torque) due to gravity on each joint of the robot arm. The processor then drives the motor 310a-h of each joint to apply a force (e.g. a torque) that will exactly oppose the calculated gravitational force. With this control strategy an operator (e.g. an operating room nurse) can push or pull any part of the robot arm to a desired position, and the part will stay in that position notwithstanding the effect of gravity on it and on any parts depending from it. A force on the arm may result in a torque about multiple joints. The control system can be programmed to decide to prioritise certain ones of the joints for neutralising the torque. In examples, some joints could be locked in position and others could move compliantly, the position of a given link or point in space could be prioritized rather than sets of joints.

Each motor 310a-h may be controlled in response to the force (e.g. torque) measured about the respective joint. When the measured force at a joint is adjusted for gravity, any remaining sensed force represents a force applied by an external force (e.g. due to a push or pull on the robot arm). In response to that force the control system may control the respective motor 310a-h so as to alter the configuration of the robot arm. For example, this may be achieved by controlling the motors 310a-h to move their respective joints 304a-g in a direction so as to reduce the measured force, and at a rate dependant on the magnitude of the measured force. In this way, the member of the bedside staff may feel that that the robot arm is moving freely in response to the force they are applying - when in fact it is the motors of the robot arm driving the movement.

The compliant mode 403 can be used to insert the surgical instrument into a port in the patient's body. That is, the compliant mode 403 may be used to insert an end effector 362 of the surgical instrument into a port 317 positioned in the patient's body. In the compliant mode 403, the surgical instrument may be positioned at the entrance of the port 317, such that the end effector 362 is concentric with the entrance of the port 317. The entrance of the port may protrude slightly from the patient's body. The end effector 362 of the surgical instrument can of course be inserted further into the port 317 in the compliant mode 403. As described herein, the port may alternatively be a natural orifice in the patient's body, such as the throat.

Referring again to Figure 3, with the robot arm in the compliant mode 403, an operator (e.g. an operating room nurse) can grasp one or both of the robot arm 301 and the surgical instrument 306. The operator can then apply external forces so as to alter the configuration of the robot arm 301 such that the elongate axis of the shaft 361 of the instrument is roughly aligned with the passageway through the hollow tube 317a of the port 317. The operator can then apply an external force (e.g. push) to the robot arm and/or the instrument such that the instrument moves roughly parallel to its elongate axis and passes into the passageway in the port 317.

The operating mode of the robot arm may also be transitioned from the compliant mode 403 to the locked mode 402 or the sleep mode 401, such as when the robot arm is being prepared for storage after a procedure has been completed.

The robot arm may be operable in a calibration mode 404. As shown in Figure 4, the operating mode of the robot arm is caused to transition from the compliant mode 403 to the calibration mode 404.

As described herein, the surgical instrument 306 can be inserted into the patient's body, e.g. via port 317. This can be performed in the compliant mode 403 or the calibration mode 404. This is because the robot arm can respond to external forces in the calibration mode 404 in the same manner as in the compliant mode 403.

In the calibration mode 404 the location of port 317 relative to the robot arm 301 is estimated. For example, the location of port 317 may be estimated relative to the wrist joints 304e-g. Specifically, a fulcrum may be determined, the fulcrum being a point about which the surgical instrument 306 pivots when inside in the patient's body. The fulcrum may be the natural rotation centre of the port 317.

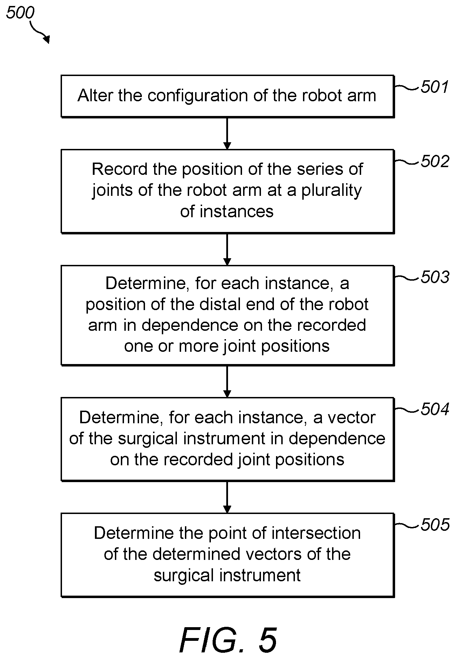

The control system (e.g. such as control system 224 shown in Figure 2) of the robot arm 301 may be capable of determining the fulcrum by means of a calibration process that is performed whilst the surgical instrument 306 is inside the port 317. Figure 5 is a flow diagram showing the steps of such a calibration process.

Whilst the surgical instrument is inside the port 317, the configuration of the robot arm is altered 501. The configuration of the robot arm 301 is altered by the application of external forces directly onto the robot arm. The robot arm can be moved generally transversely to the shaft 361 of the instrument 306. This causes the port 317 to apply a lateral force on the instrument shaft 316. That force is accommodated by motion about the joint 304f. As the configuration of the robot arm is being altered, the position sensors 307a-h record the position of each joint of the robot arm. The position sensors 307a-h record 502 the positions of each joint of the robot arm at a plurality of instances. That is, the position sensors 307a-h record the positions of each joint of the robot arm at a plurality of points in time. Position information may be recorded irregularly or at predetermined intervals, e.g. every 0.5 seconds. The position sensors provide the recorded position information to the control system. The control system uses this received information to determine: (a) the position of the distal end of the robot arm relative to the base and (b) the vector of the instrument shaft 361 relative to the distal end of the robot arm. Position (a) and vector (b) may be termed a data pair. The control system may determine a data pair for each instance at which the position sensors recorded position information. That is, for each instance, the control system determines a position of the distal end of the robot arm 503 in dependence on the recorded one or more joint positions. In addition, for each instance, the control system determines a vector of the surgical instrument from the determined position of the distal end of the second robot arm 504 in dependence on the recorded joint positions. Since the axis of the instrument shaft 361 passes through the passageway of the port 317, the passageway of the port lies along that vector. As the distal end of the robot arm is moved, the control system calculates multiple pairs of distal end positions and instrument shaft vectors. Those vectors all converge, from their respective distal end position, on the natural rotation centre of the passageway of the port 317. By collecting a series of those data pairs and then solving for the mean location where the instrument shaft vectors converge the control system estimates the location of the port relative to the robot arm. That is, the control system determines the point of intersection of the determined vectors of the surgical instrument 505 so as to determine the fulcrum. The control system then stores the determined fulcrum in non-transient form in memory for later use.

During step 501, the configuration of the robot arm may be altered such that the distal end of the robot arm is moved in two dimensions: e.g. with (i) components parallel to a direction that is transverse to the instrument shaft 361 and also with (ii) components orthogonal to that direction but transverse to the instrument shaft 361. To do this, the operator (e.g. a member of the bedside team) may gyrate the distal end of the robot arm about a point generally aligned with the natural axis of the hollow tube 317a of the port.

The number of data pairs determined in step 502, and used in steps 503 to 505, to determine the fulcrum with acceptable precision depends on factors such as the accuracy of the robot arm's position sensors and the extent to which the operator moves the arm laterally during the calibration process. The control system may determine that the fulcrum has been estimated adequately once sufficient coherent measurements have been gathered such that the variance between estimates of the fulcrum derived using successive measurements has reduced below a predefined level.

After determining the fulcrum, the robot arm may be operable in an instrument adjust mode 405. As shown in Figure 4, the operating mode of the robot arm may be caused to transition from the calibration mode 404 to the instrument adjust mode 405. The operating mode of the robot arm may be caused to transition from the surgical mode 406 to the instrument adjust mode 405.

In the instrument adjust mode 405, the configuration of the robot arm can be altered in response to external forces in the same manner as in the compliant mode 403, with the exception that the configuration of the robot arm is constrained such that an intersection is maintained between the instrument shaft 361 and the determined fulcrum. The instrument adjust mode 405 can be used to adjust the position of the instrument within the patient's body. For example, instrument adjust mode 405 can be used to position the instrument in an optimal position to begin a procedure. For example, as described herein, the in the compliant mode 403, the surgical instrument may be positioned at the entrance of the port 317, such that the end effector 362 is concentric with the entrance of the port 317. The instrument adjust mode 405 may be used to assist insertion of the instrument further into the patient's body (e.g. towards an intended surgical site). In this example, the control system (such as control system 224 in Figure 2) uses the determined position of the fulcrum to control the arm to adopt a configuration in which the instrument is generally aligned with the port passage. Then, an operator (e.g. a member of the bedside team, such as an operating room nurse) can insert the instrument further through the port by applying external forces to the robot arm. Whilst the instrument is being inserted further through the port, the control system controls the robot arm such that the shaft of the instrument intersects the fulcrum. That is, an intersection between the instrument and the fulcrum is maintained.

After determining the fulcrum, the robot arm is operable in a surgical mode 406. As shown in Figure 4, the operating mode of the robot arm may be caused to transition directly from the calibration mode 404 to the surgical mode 406, or from the instrument adjust mode 405 to the surgical mode 406.

In the surgical mode 406, the configuration of the robot arm may be remotely controlled in response to inputs received at a remote surgeon console (such as remote surgeon console 220 shown in Figure 2). A surgeon may provide inputs to the remote console 220. The remote surgeon console comprises one or more surgeon input devices 223. For example, these may take the form of a hand controller and/or foot pedal.

In the surgical mode 406 the operator (e.g. a surgeon) uses the remote surgeon console to signal a desired position of the end effector 362. The control system (such as control system 224 shown in Figure 2) determines a configuration of the joints of the robot arm that will result in the end effector 362 being placed in that position. There may be multiple configurations of the robot arm that will result in the end effector 362 being placed in the desired position. The control system may select between those configurations based on an algorithm that seeks to avoid collisions between the robot arm and other objects known to the control system to be close to the robot arm, or that seeks to minimise the amount of movement of the joints to reach the new configuration. Once the control system has selected a new configuration it signals the joints 304a-g to adopt the states required to bring the arm into that configuration. In this way, in the surgical mode 406 the operator (e.g. a surgeon) can signal the end effector 362 to move to a desired location.

The control system uses the determined fulcrum to assist in controlling the configuration of the robot arm when the robot arm is operating in the surgical mode 406. The control system is configured, e.g. by means of the software stored in memory, to select a configuration of the arm for which both (i) the end effector 362 is at the desired position and (ii) the shaft 361 of the instrument 306 passes through the determined fulcrum, and to move the arm to that configuration. In that way the end effector 362 can be provided at the desired position with relatively little disruption to the outer tissues of the patient.

The surgical mode 406 may comprise an engaged surgical mode and a disengaged surgical mode. In the engaged surgical mode, the configuration of the robot arm and the surgical instrument is controlled by a remote surgeon console as described herein. In the disengaged surgical mode, the configuration of the robot arm and surgical instrument is controllable by a remote surgeon console. That is, in the disengaged surgical mode the fulcrum about which the surgical instrument pivots when the configuration of the robot arm is altered is known - and thus it is possible for the configuration of the robot arm and the surgical instrument to be controlled by a remote surgeon console. However, in the disengaged surgical mode, the configuration of the surgical instrument may temporarily be locked or maintained. For example, a surgeon may opt to place a robot arm in the disengaged surgical mode in order to take a rest, or such that they can focus their attention of the control of a different robot arm (e.g. during a particularly difficult part of a procedure). The surgeon may control the transition between the engaged and disengaged surgical modes - e.g. via an interface on the remote surgeon console, or by instructing a member of the operating room staff to interact with an interface on the robot arm itself.

When operating in the surgical mode 406, certain portions of the robot arm may exhibit compliant-like behaviour. For example, the configuration of the elbow joint 304d may be capable of being altered in response to external forces in the manner described herein, so long as the configuration of the instrument 306 is not affected. Enabling such compliant-like behaviour whilst the robot arm is operating in the surgical mode allows, for example, an operator of the robotic surgical system to move the elbow of the robot arm (e.g. so that they can access the patient during the procedure). In order to implement such compliant-like behaviour the control system may define an allowed area or volume for one or more parts the robot (e.g. the set of wrist joints 304e-g), such that the movement of those parts in response to externally applied forces is confined within that allowed area or volume. The allowed area or volume is defined such that movements within that area or volume in response to externally applied forces do not cause the configuration of the instrument 306 to be affected.

In examples where the robot arm comprises an orientation interface, the control system may also use the identified common direction to translate inputs at the remote surgeon console into suitable control signals for controlling the configuration of the robot arm when operating in the surgical mode 406.

The robot arm may also be operable in an instrument retract mode 406. As shown in Figure 4, the operating mode of the robot arm may be caused to transition from the surgical mode 406 to the instrument retract mode 407.

The instrument retract mode 407 is engaged in order to remove the instrument 306 from a patient's body (e.g. at the end of a procedure). In the instrument retract mode 407, the control system controls the motors 310a-h to reconfigure the robot arm so as to cause the instrument 306 to be retracted from the port along the longitudinal axis of the instrument. The longitudinal axis of the instrument may be co-axial with the instrument shaft 361. The control system can achieve this by controlling the motors 310a-h so as to permit the robot arm to be reconfigured by the action of external forces applied to the robot arm, similar to the compliant mode 403. That is, in the instrument retract mode 407 the control system enables the configuration of the robot arm to be altered in response to external forces, but limits the freedom of motion of the robot arm such that the surgical instrument can only move linearly in directions parallel and/or co-axial with its longitudinal axis and away from the patient's body. In other words, an external force applied to the robot arm parallel with the shaft 361 of the instrument 306 and in a direction away from the patient's body, causes the instrument to be extracted from the patient's body. On detecting external force applied in this direction, the control system responds signalling the motors 310a-h of the appropriate joints to drive the joints to move in the direction that the external force is applied. The force of gravity on each joint is opposed as described above with respect to the compliant mode 403. In this way, an operator (e.g. a member of the bedside team, such as an operating room nurse) can manually push or pull the robot arm in a direction away from the patient's body, and the robot arm will respond by moving in that direction. Thus, the robot arm provides the sensation to the operator of moving freely under their push or pull to withdraw the instrument from the patient's body.

The control system detects external forces applied to the robot arm by means of force sensors 307a-h. The control system uses this sensor input to determine if an applied external force is acting along and/or parallel to the longitudinal axis of the instrument and away from the patient's body. In examples where the instrument extends along an axis coincident with the rotation axis of joint 304g, the control system may use the sensor input to determine if an applied external force is coincident (and therefore also inherently parallel) with the longitudinal axis of the instrument and away from the patient's body. In examples where the instrument extends linearly parallel with the rotation axis of the terminal joint 304g of the robot arm (but not necessarily co-axial with that rotation axis), the control system may use the sensor input to determine if an applied external force is parallel with the longitudinal axis of the instrument and away from the patient's body.

In order to make this determination, the control system resolves the detected applied forces, and signals the motors 310a-h to drive the robot arm based only on the components of the forces parallel with the longitudinal axis of the instrument. In the instrument retract mode 407, components of applied external forces in a direction transverse to the longitudinal axis of the instrument are not acted upon. External forces applied in such directions are thereby resisted. Further, components of applied external forces acting along the longitudinal axis of the instrument towards the patient's body are not acted upon. External forces applied in this direction are thereby resisted.

The operating mode of the robot arm may be transitioned from the instrument retract mode 407 to any of the complaint mode 403, the calibration mode 404, or the instrument adjust mode 405.

As described herein with reference to Figure 3, robot arm 301 may comprise one or more interfaces 370, 371. Interfaces 370, 371 may be, for example, a button or set of buttons accessible by a member of the bedside team (e.g. an operating room nurse). Interfaces 370, 371 can be actuated to transition between the operating modes described with reference to Figure 4.

Referring again to Figure 3, interface 370 may be positioned in a more distal position than interface 371. That is, interface 370 may be positioned closer to the surgical instrument 306 than interface 371. For example, interface 370 may be positioned on or near to the wrist joints 304e-g, whilst interface 371 is positioned on or near elbow joints 304d. The robot arm 301 may be configured such that an operator interaction with the more distal interface 370 causes the operating mode of the robot arm to transition "towards" the surgical mode 406. The operating mode transitions considered to be "towards" the surgical mode 406 are labelled 370 in Figure 4. For example, transitions "towards" the surgical mode 406 include: transitions from the sleep mode 401 to the locked mode 401 or the compliant mode 403; a transition from the locked mode 402 to the compliant mode 403; a transition from the compliant mode 403 to the calibration mode 404; and transitions from the calibration mode 404 to the instrument adjust mode 405 or the surgical mode 406. The robot arm 301 may be configured such that an operator interaction with the less distal interface 371 causes the operating mode of the robot arm to transition "away from" the surgical mode 406. The operating mode transitions considered to be "away from" the surgical mode 406 are labelled 371 in Figure 4. For example, transitions "away from" the surgical mode 406 include: a transition from the surgical mode 406 to the instrument adjust mode 405; transitions from the instrument retract mode 407 to any of the compliant mode 403, calibration mode 404 or instrument adjust mode 405; transitions from the compliant mode 403 to the locked mode 402 or the sleep mode 401; and a transition from the locked mode 402 to the sleep mode 401. For example, if a robot arm were to be operating in the compliant mode 403, an interaction with: (i) the more distal interface 370 may cause the operating mode to transition to the calibration mode 404, and (ii) the less distal interface 371 may cause the operating mode to transition to the locked mode 402. In this manner, the selection of the next operating mode is more intuitive to the operator.

One or more conditions may be associated with each operating mode. These conditions may determine whether that mode can be accessed. For example, the surgical mode may require that the fulcrum has been determined and that the instrument is inserted by at least a predetermined distance within the patient's body (e.g. to ensure that the instrument is observable by the surgeon through an endoscope within the patient's body). Under certain conditions (e.g. under safety alarm situations) some modes may not be available.

### Re-configuring a surgical robotic system

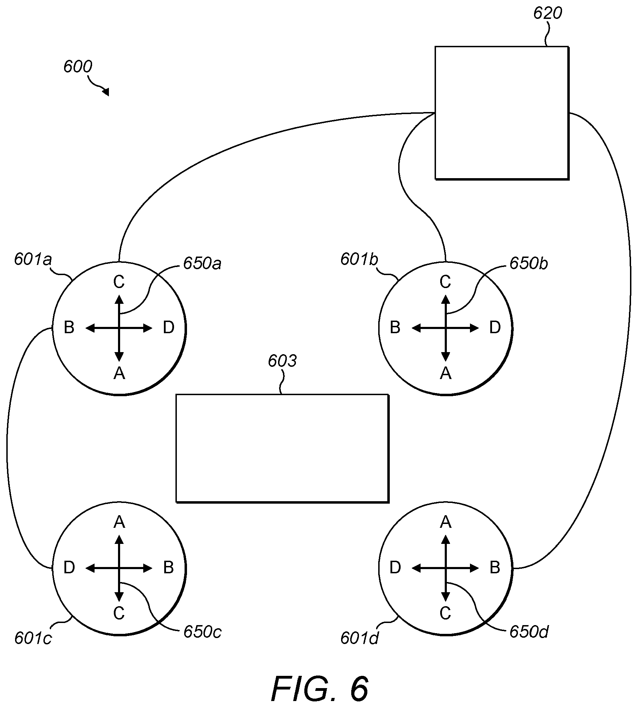

The re-configuration of a surgical robotic system will be described with reference to Figure 6. Figure 6 shows a plan view of a surgical robotic system. Surgical robotic system 600 comprises robot arms 601a, 601b, 601c, 601d. For simplicity, the robot arm linkages and joints of each of robot arms 601a-d are not shown in Figure 6. Each robot arm 601a-d may comprise equivalent features to robot arm 301 described with reference to Figure 3. Although four robot arms 601a-d are shown in Figure 6, it is to be understood that a surgical robotic system configured in accordance with the principles described herein may comprise any number of robot arms.

Robot arms 601a-d are positioned about operating table 603. For simplicity, no patient is shown in Figure 6. Figure 6 shows a robot arm 601a-d positioned on each corner of operating table 603, but is to be understood that robot arms 601a-d may be positioned in any other arrangement.

Figure 6 also shows remote surgeon console 620. Control system 624 is not shown in Figure 6 - but could be located in any of the positions described previously herein. It is to be understood that the control system of the surgical robotic system is configured to reconfigure the surgical robotic system by controlling the operation of each robot arm in the surgical robotic system - i.e. in accordance with the various operating modes described with reference to Figure 4. Robot arms 601a-d may be connected to console 620 by wired, or wireless, connections. Said connections may provide control signals, and optionally power, from the console 620 to each robot arm 601a-d. One or more of the robot arms 601a-d may be directly connected to a power source (i.e. not connected to a power source via the console) and/or may be powered by a local power source, such as a battery. Said connections may also provide feedback from the robot arm 601a-d to the console 620. A suitable wired connection for communicating control signals is an ethernet field bus.

As shown in Figure 6, robot arms 601b and 601d are connected to console 620 by direct connections. Robot arm 601a is also connected to console 620 by a direct connection. Robot arm 601c is not connected to console 620 by a wired connection, but rather is connected to robot arm 601a which itself is connected to console 620 by a direct connection. Robot arms 601a and 601c and 130 may be considered to be part of a "daisy chain". All robot arms in a surgical robotic system may be connected to a console by direct connections, or all robot arms may be part of a "daisy chain", or any mixture of direct connections and "daisy chain" connections may be used (e.g. as shown in Figure 6).

At any point in time, each robot arm 601a-d in surgical robotic system 600 may be operating in a different one of the modes shown in Figure 5. At any point in time, each robot arm in a surgical robotic system may be operating in the same mode.

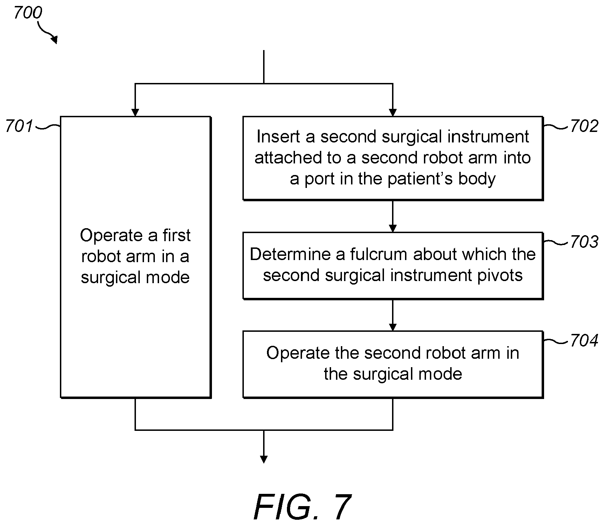

Figure 7 shows a flow diagram for reconfiguring a surgical robotic system in accordance with the principles described herein. A robot arm may be added to a surgical robotic system whilst a robot arm already part of the surgical robotic system is operating in the surgical mode 406. For example, with reference to Figure 6, a procedure may be ongoing using only first robot arm 601a. That is, the surgical instruments of robot arms 601b-d may not initially be inserted into the patient's body. The first robot arm 601a may be operating in the surgical mode 406. The second robot arm 601b may be added to surgical robotic system 600 whilst the first robot arm 601a is operating in a surgical mode 406. Adding a second robot arm 601b to a surgical robotic system involves inserting 702 the surgical instrument it carries ("the second surgical instrument") into a port in the patient's body and configuring that robot arm such that it can be operated in a surgical mode 406. That is, the fulcrum about which the second surgical instrument pivots whilst inside the port is determined 703 (as described herein with reference to the calibration mode 404). The second robot arm 601b can then be operated 704 in the surgical mode in which the configuration of the second robot arm and the second surgical instrument is controlled by the remote surgeon console 620 whilst maintaining an intersection between the second surgical instrument and the determined fulcrum (as described herein with reference to the surgical mode 406). The first robot arm 601a may be continually operated in the surgical mode whilst the second robot arm 601b is being added to surgical robotic system 600. That is, the second robot arm 601b may be added to surgical robotic system 600 without interrupting the operation of the first robot arm 601a in the surgical mode 406. In other words, the operation 701 of the first robot arm 601a in the surgical mode 406 and the addition 702, 703, 704 of the second robot arm 601b to the surgical robotic system 600 may be performed concurrently. The first robot arm 601a may be operated 701 in the engaged surgical mode or the disengaged surgical mode, or sequentially in the engaged and disengaged surgical modes, as described herein whilst the second robot arm 601b is being added 702, 703, 704 to the surgical robotic system 600. Adding the second robot arm 601b to the surgical robotic system may involve operating the second robot arm 601b in any one of more of the sleep mode 401, locked mode 402, compliant mode 403, calibration mode 404 and instrument adjust mode 405 as described with reference to Figure 4, prior to operating the second robot arm 601b in the surgical mode 406.

As described herein, the robot arm may be supported by a moveable support structure. Prior to adding a robot arm to a surgical robotic system, the robot arm may be moved into a position adjacent to the patient. A robot arm may be moved from a position remote from the patient into a position adjacent to the patient. For example, a spare robot arm may be provided in an operating room, or external to the operating room, for use when required (e.g. for any of the reasons previously given herein). Alternatively, a robot arm may be moved from a position adjacent to the patient into a different position adjacent to the patient (e.g. for the reasons given in the preceding paragraph). After a robot arm has been moved into a position adjacent to the patient, it may be added to the surgical robotic system. The robot arm could be moved into a position adjacent to the patient in any of the sleep mode 401, locked mode 402 or compliant mode 403.

As described herein with reference to Figure 3, each of robot arms 601a-d may comprise an orientation interface 650a-d. The orientation interface 650a-d of each robot arm 601a-d may be, for example, a button or set of buttons accessible by a member of the bedside team (e.g. an operating room nurse). Although the orientation interfaces 601a-d shown in Figure 6 are shown indicating four directions, it is to be understood that any number of directions may be indicated.

The orientation interface 650a-d of each robot arm 601a-d can be used to identify a common direction for the robot arms 601a-d of surgical robotic system 600. In an example, a direction may be selected using each orientation interface 650a-d such that all of the selected directions point in the same direction. For example, with reference to Figure 6, direction A may be selected for robot arms 601a and 601b, and direction C may be selected for robot arms 601c and 601d.

The common direction may be used by the robotic surgical system 600 in order to translate inputs to the remote console 620 into suitable control signals for one or more of the robot arms 601a-d, when those robot arms are operating in the surgical mode 406. The common direction may be identified in any of the sleep mode 401, locked mode 402 or compliant mode 403.

The robot arms may not have orientation interfaces. For example, in some surgical robotic systems the robot arms are positioned in predetermined locations and orientations. For example, a jig may be provided in the operating room which enables each robot arm to be accurately positioned in a predetermined location and orientation, a camera based tracking system may be provided for detecting the relative location and orientation of the robot arms, or locating devices may be within the robot arms for detecting the relative location and orientation of the robot arms.

A robot arm may need not be moved prior to being added to a surgical robotic system. That is, a robot arm may already be located in a position adjacent to the patient. For example, with reference to Figure 6, robot arms 601a-c may be part of a surgical robotic system. Robot arm 601d may be located in the position shown in Figure 6, but may not be being used as part of the surgical robotic system. That is, robot arm 601d may be a redundant robot arm. If required during a procedure, robot arm 601d could be added to the surgical robotic system whilst one or more of robot arms 601a-c were operating in the surgical mode 406.

In an example, a new robot arm may be added to a surgical robotic system so as to increase the capabilities of that surgical robotic system. For example, a user of the surgical robotic system (e.g. a surgeon) may desire an additional surgical instrument to aid in a procedure - and so a new robot arm carrying that instrument may be added into the surgical robotic system.

In other examples, a new robot arm may be added to a surgical robotic system in exchange for a robot arm already part of the surgical robotic system. For example, such an exchange may be performed if a robot arm already part of the surgical robotic system develops a fault. That is, a robot arm already part of the surgical robotic system may be removed prior to adding a new robot arm, or a new robot arm may be added to the surgical robotic system prior to removing a robot arm. In order to remove the faulted robot arm from the system, the instrument retract mode described herein may be used. In other words, a robot arm of the surgical robotic system may be replaced by a different robot arm. Examples of faults for which a robot arm may be replaced include mechanical faults (e.g. the failure of a drive cable under mechanical stress), software faults (e.g. the introduction of a software bug), a loss of power from the console, a loss of communication with the console, a loss of communication with the instrument, a casing of the robot arm exceeding predetermined temperature (e.g. caused by a motor of the robot arm overheating), other issues such as a local battery level running low, tracking errors by the motors (e.g. motor slip), problems in the moveable support structure (e.g. a broken wheel, or a brake malfunction) or the case of rail mounted arms, mechanical problems with the rail-arm attachment, or any other fault.

In another example, a robot arm may be removed from the surgical robotic system and then be added back into the same surgical robotic system. In these examples, a robot arm may be removed in order that a maintenance task can be performed (e.g. a fault can be repaired, and/or an alarm can be reset) before that robot arm is added back into the system, or a robot arm may be removed in order that it can be moved into a different location relative to the operating table (e.g. so as to provide better access to a surgical site or avoid collisions with other robot arms) before being added back into the surgical robotic system.

The robot arm described herein could be for purposes other than surgery. For example, the port could be an inspection port in a manufactured article such as a car engine and the robot could control a viewing instrument for viewing inside the engine.

## Claims

1. A control system (224) of a surgical robotic system (200), the surgical robotic system (200) comprising a first robot arm (201a) and a second robot arm (201b), each of the first and second robot arms (201a, 201b) comprising a series of joints (204) by which the configuration of that robot arm (201a, 201b) can be altered, the series of joints (204) extending from a base (209) at a proximal end of the robot arm (201a, 201b) to an attachment for a surgical instrument (206) at a distal end of the robot arm (201a, 201b), the control system (224) being configured to reconfigure the surgical robotic system (200) by:
controlling the first robot arm to operate in a surgical mode (406) in which a first surgical instrument (206) attached to that first robot arm (201a) is inside a patient's body and the configuration of the first robot arm and first surgical instrument is controlled in response to inputs received at a remote surgeon console; and
**characterised in that** the control system (224) is configured to reconfigure the surgical robotic system (200) by:
whilst the first robot arm (201a) is operating in the surgical mode (406):
(i) controlling the second robot arm (201b) so as to permit a second surgical instrument (206) attached to the second robot arm (201b) to be inserted into a port (317) in the patient's body;
(ii) determining a fulcrum about which the second surgical instrument (206) pivots when the configuration of the second robot arm (201b) is altered whilst the second surgical instrument (206) is inside the port (317); and
(iii) controlling the second robot arm (201b) to operate in the surgical mode (406) in which the second surgical instrument (206) attached to that second robot arm (201b) is inside the patient's body and the configuration of the second robot arm (201b) and second surgical instrument (206) is controlled in response to inputs received at the remote surgeon console (220) whilst maintaining an intersection between the second surgical instrument (201b) and the determined fulcrum.

2. The control system (224) as claimed in claim 1, wherein the fulcrum is determined by:
controlling the second robot arm (201b) so as to enable its configuration to be altered in response to external forces whilst the second surgical instrument (206) is inside the port (317); and
determining the fulcrum, the fulcrum being the point about which the surgical instrument (206) of the second robot arm (201b) pivots whilst inside the port (317).

3. The control system (224) as claimed in claim 2, wherein the second robot arm (201b) further comprises one or more force sensors (308a-h) configured to sense external forces at one or more joints (204) of the series of joints (204) of the second robot arm (201b), and one or more motors (310a-h) configured to drive one or more joints (204) of the series of joints of the second robot arm (201b), and the control system (224) is further configured to:
control the one or more motors (310a-h) so as to drive one or more joints (204) of the series of joints (204) of the second robot arm (201b) in dependence on external forces sensed by the one or more force sensors (308a-h) so as to alter the configuration of the second robot arm (201b).

4. The control system (224) as claimed in any preceding claim, wherein the second robot arm (201b) further comprises one or more position sensors (307a-h) configured to sense the position of one or more joints (204) of the series of joints (204) of the second robot arm (201b) and to record the position of one or more joints (204) of the series of joints (204) of the second robot arm (201b) at a plurality of instances whilst the configuration of the second robot arm (201b) is being altered, and the control system (224) further is configured to:
determine, for each instance, a position of the distal end of the second robot arm (201b) in dependence on the respective recorded one or more joint positions;
determine, for each instance, a vector of the second surgical instrument (201b) from the determined position of the distal end of the second robot arm (201b) in dependence on the respective recorded one or more joint positions; and
determine the point of intersection of the determined vectors of the second surgical instrument (201b) so as to determine the fulcrum.

5. The control system (224) as claimed in any preceding claim, the control system (224) being further configured to:
determine the fulcrum when controlling the second robot arm (201b) to operate in a calibration mode (404); and
control the second robot arm (201b) to transition from operating in the calibration mode (404) to operating in the surgical mode (406), and
optionally wherein the second robot arm (201b) further comprises a more distal interface and a less distal interface, and the control system (224) is further configured to:
control the second robot arm (201b) to transition from operating in the calibration mode (404) to operating in the surgical mode (406) in response to an operator interaction with the more distal interface (370).

6. The control system (224) as claimed in claim 5, the control system (224) being further configured to:
control the second robot arm (201b) so as to permit the second surgical instrument (206) to be inserted into the port (317) by controlling the second robot arm (201b) to operate in a compliant mode (403) in which the configuration of the second robot arm (201b) can be altered in response to external forces; and
control the second robot arm (201b) to transition from operating in the compliant mode (403) to operating in the calibration mode (404), and
optionally the control system being further configured to:
control the second robot arm (201b) to transition from operating in the compliant mode (403) to operating in the calibration mode (404) in response to a user interaction with the more distal interface (370).

7. The control system (224) as claimed in claim 5 or 6, the control system (224) being further configured to:
after determining the fulcrum, control the second robot arm (201b) to operate in an instrument adjust mode (405) in which the configuration of the second robot arm (201b) can be altered in response to external forces but is constrained such that an intersection is maintained between the second surgical instrument (206) and the determined fulcrum.

8. The control system (224) as claimed in claim 7, the control system (224) being further configured to:
control the second robot arm (201b) to transition from operating in the calibration mode (404) to operating in the instrument adjust mode (405);
control the second robot arm (201b) to transition from operating in the instrument adjust mode (405) to operating in the surgical mode (406); and
optionally, control the second robot arm to transition from operating in the surgical mode (406) to operating in the instrument adjust mode (405).

9. The control system (224) as claimed in any preceding claim, wherein each of the first robot arm (201a) and second robot arm (201b) further comprise an orientation interface (350), and the control system (224) is further configured to:
receive an input identifying a common direction in response to an operator indicating a direction using the orientation interface (350) of the first robot arm (201a) and indicating a corresponding direction using the orientation interface (350) of the second robot arm (201b).

10. The control system (224) as claimed in claim 9, wherein, in the surgical mode (406), the second robot arm (201b) is remotely controlled by the control system (224) being configured to:
receive inputs relating to the second robot arm (201b) to the remote console (220);
convert the inputs into control signals for the second robot arm (201b) in dependence on the determined fulcrum and the identified common direction; and
control one or more joints (204) of the series of joints (204) of the second robot arm (201b) in dependence on the control signals so as to control the configuration of the second robot arm (201b).

11. The control system (224) as claimed in any preceding claim, wherein the surgical robotic system (200) comprises a third robot arm (601c) comprising a series of joints by which the configuration of that robot arm can be altered, the series of joints extending from a base at a proximal end of the robot arm to an attachment for a surgical instrument at a distal end of the robot arm, and the control system (224) is further configured to:
whilst controlling the first robot arm (201a) to operate in the surgical mode (406) and prior to permitting the second surgical instrument (201b) to be inserted into the port (317), control the third robot arm (201c) so as to permit a third surgical instrument attached to the third robot arm to be retracted from the patient's body, and/or
the control system (224) being further configured to permit the third surgical instrument to be retracted from the patient's body by:
enabling the configuration of the third robot arm (601c) to be altered in response to external forces, the freedom of motion of the third robot arm (601c) being limited such that the third surgical instrument can only move linearly in directions co-axial with the longitudinal axis of the third surgical instrument and away from the patient's body.

12. The control system (224) as claimed in claim 11, wherein the third robot arm (601c) further comprises one or more force sensors configured to sense external forces at one or more joints of the series of joints of the third robot arm (601c), and one or more motors configured to drive one or more joints of the series of joints of the third robot arm (601c), and the control system (224) is further configured to:
resolve external forces sensed by the one or more force sensors so as to determine the components of the forces parallel with the longitudinal axis of the third surgical instrument and away from the patient's body; and
control the one or more motors so as to drive one or more joints of the series of joints of the third robot arm in dependence on the components of the forces parallel with the longitudinal axis of the third surgical instrument so as to alter the configuration of the third robot arm.

13. The control system (224) as claimed in any preceding claim, wherein the control system (224) is further configured to; whilst controlling the first robot arm (201a) to operate in the surgical mode (406) and prior to permitting the second surgical instrument (206) to be inserted into the port (317), control the second robot arm (201b) so as to permit the second surgical instrument (206) to be retracted from the patient's body; and control the second robot arm (201b) so as to permit the second surgical instrument (206) to be inserted into the patient's body after a maintenance task has been performed on the second robot arm (201b), and
optionally the control system (224) being further configured to permit the second surgical instrument (206) to be retracted from the patient's body by:
enabling the configuration of the second robot arm (201b) to be altered in response to external forces, the freedom of motion of the second robot arm (201b) being limited such that the second surgical instrument (206) can only move linearly in directions parallel with the longitudinal axis of the second surgical instrument (206).

14. The control system (224) as claimed in claim 13, wherein the second robot arm (201b) further comprises one or more force sensors (308a-h) configured to sense external forces at one or more joints (204) of the series of joints (204) of the second robot arm (201b), and one or more motors (310a-h) configured to drive one or more joints (204) of the series of joints (204) of the second robot arm (201b), and the control system (224) is further configured to:
resolve external forces sensed by the one or more force sensors (308a-h) so as to determine the components of the forces parallel with the longitudinal axis of the second surgical instrument (206); and
control the one or more motors (310a-h) so as to drive one or more joints of the series of joints of the second robot arm (201b) in dependence on the components of the forces parallel with the longitudinal axis of the second surgical instrument (206) so as to alter the configuration of the second robot arm (201b).

## Patentansprüche

1. Steuersystem (224) eines chirurgischen Robotersystems (200), wobei das chirurgische Robotersystem (200) einen ersten Roboterarm (201a) und einen zweiten Roboterarm (201b) umfasst, wobei jeder von dem ersten und dem zweiten Roboterarm (201a, 201b) eine Reihe von Gelenken (204) umfasst, durch die die Konfiguration des Roboterarms (201a, 201b) verändert werden kann, wobei sich die Reihe von Gelenken (204) von einer Basis (209) an einem proximalen Ende des Roboterarms (201a, 201b) zu einer Befestigung für ein chirurgisches Instrument (206) an einem distalen Ende des Roboterarms (201a, 201b) erstreckt, wobei das Steuersystem (224) dazu konfiguriert ist, das chirurgische Robotersystem (200) umzukonfigurieren durch:
Steuern des ersten Roboterarms, um in einem chirurgischen Modus (406) zu arbeiten, in dem ein erstes chirurgisches Instrument (206), das an dem ersten Roboterarm (201a) angebracht ist, sich innerhalb des Körpers eines Patienten befindet, und die Konfiguration des ersten Roboterarms und des ersten chirurgischen Instruments in Reaktion auf Eingaben gesteuert wird, die an einer entfernten Chirurgenkonsole empfangen werden; und
**dadurch gekennzeichnet, dass** das Steuersystem (224) konfiguriert ist, das chirurgische Robotersystem (200) umzukonfigurieren durch:
während der erste Roboterarm (201a) in dem chirurgischen Modus (406) arbeitet:
(i) Steuern des zweiten Roboterarms (201b), um zu ermöglichen, dass ein zweites chirurgisches Instrument (206), das an dem zweiten Roboterarm (201b) angebracht ist, in einen Port (317) im Körper des Patienten eingeführt wird;
(ii) Ermitteln eines Drehpunkts, um den das zweite chirurgische Instrument (206) schwenkt, wenn die Konfiguration des zweiten Roboterarms (201b) geändert wird, während sich das zweite chirurgische Instrument (206) innerhalb des Ports (317) befindet; und
(iii) Steuern des zweiten Roboterarms (201b), um in dem chirurgischen Modus (406) zu arbeiten, in dem sich das zweite chirurgische Instrument (206), das an diesem zweiten Roboterarm (201b) angebracht ist, innerhalb des Körpers des Patienten befindet, und die Konfiguration des zweiten Roboterarms (201b) und des zweiten chirurgischen Instruments (206) in Reaktion auf Eingaben gesteuert wird, die an der entfernten Chirurgenkonsole (220) empfangen werden, während ein Schnittpunkt zwischen dem zweiten chirurgischen Instrument (201b) und dem ermittelten Drehpunkt beibehalten wird.

2. Steuersystem (224) nach Anspruch 1, wobei der Drehpunkt ermittelt wird durch:
Steuern des zweiten Roboterarms (201b), um zu ermöglichen, dass seine Konfiguration in Reaktion auf externe Kräfte geändert wird, während sich das zweite chirurgische Instrument (206) innerhalb des Ports (317) befindet; und
Ermitteln des Drehpunkts, wobei der Drehpunkt der Punkt ist, um den das chirurgische Instrument (206) des zweiten Roboterarms (201b) schwenkt, während es sich innerhalb des Ports (317) befindet.

3. Steuersystem (224) nach Anspruch 2, wobei der zweite Roboterarm (201b) ferner einen oder mehrere Kraftsensoren (308a-h) umfasst, die konfiguriert sind, externe Kräfte an einem oder mehreren Gelenken (204) der Reihe von Gelenken (204) des zweiten Roboterarms (201b) zu erfassen, und einen oder mehrere Motoren (310a-h), die konfiguriert sind, ein oder mehrere Gelenke (204) der Reihe von Gelenken des zweiten Roboterarms (201b) anzutreiben, und wobei das Steuersystem (224) ferner konfiguriert ist zum:
Steuern des einen oder der mehreren Motoren (310a-h), um ein oder mehrere Gelenke (204) der Reihe von Gelenken (204) des zweiten Roboterarms (201b) in Abhängigkeit von durch den einen oder die mehreren Kraftsensoren (308a-h) erfassten externen Kräften anzutreiben, um die Konfiguration des zweiten Roboterarms (201b) zu ändern.

4. Steuersystem (224) nach einem der vorhergehenden Ansprüche, wobei der zweite Roboterarm (201b) ferner einen oder mehrere Positionssensoren (307a-h) umfasst, die so konfiguriert sind, dass sie die Position von einem oder mehreren Gelenken (204) der Reihe von Gelenken (204) des zweiten Roboterarms (201b) erfassen und die Position von einem oder mehreren Gelenken (204) der Reihe von Gelenken (204) des zweiten Roboterarms (201b) zu einer Mehrzahl von Zeitpunkten aufzeichnen, während die Konfiguration des zweiten Roboterarms (201b) verändert wird, und wobei das Steuersystem (224) ferner konfiguriert ist zum:
Ermitteln, für jede Instanz, einer Position des distalen Endes des zweiten Roboterarms (201b) in Abhängigkeit von der jeweiligen aufgezeichneten einen oder mehreren Gelenkpositionen;
Ermitteln, für jede Instanz, eines Vektors des zweiten chirurgischen Instruments (201b) aus der ermittelten Position des distalen Endes des zweiten Roboterarms (201b) in Abhängigkeit von der jeweiligen aufgezeichneten einen oder mehreren Gelenkpositionen; und
Ermitteln des Schnittpunkts der ermittelten Vektoren des zweiten chirurgischen Instruments (201b), um den Drehpunkt zu ermitteln.

5. Steuersystem (224) nach einem der vorhergehenden Ansprüche, wobei das Steuersystem (224) ferner konfiguriert ist zum:
Ermitteln des Drehpunkts, wenn der zweite Roboterarm (201b) so gesteuert wird, dass er in einem Kalibriermodus (404) arbeitet; und
Steuern des zweiten Roboterarms (201b), sodass er von dem Betrieb im Kalibriermodus (404) zu dem Betrieb im chirurgischen Modus (406) übergeht, und
optional, wobei der zweite Roboterarm (201b) ferner eine distalere Schnittstelle und eine weniger distale Schnittstelle umfasst, und wobei das Steuersystem (224) ferner konfiguriert ist zum:
Steuern des zweiten Roboterarms (201b), sodass dieser in Reaktion auf eine Bedienerinteraktion mit der distaleren Schnittstelle (370) von dem Betrieb im Kalibriermodus (404) zu dem Betrieb im chirurgischen Modus (406) übergeht.

6. Steuersystem (224) nach Anspruch 5, wobei das Steuersystem (224) ferner konfiguriert ist zum:
Steuern des zweiten Roboterarms (201b), um das Einführen des zweiten chirurgischen Instruments (206) in den Port (317) zu ermöglichen, indem der zweite Roboterarm (201b) so gesteuert wird, dass er in einem nachgiebigen Modus (403) arbeitet, in dem die Konfiguration des zweiten Roboterarms (201b) in Reaktion auf externe Kräfte geändert werden kann; und
Steuern des zweiten Roboterarms (201b), sodass er von dem Betrieb im nachgiebigen Modus (403) zu dem Betrieb im Kalibriermodus (404) übergeht, und
optional, wobei das Steuersystem ferner konfiguriert ist zum:
Steuern des zweiten Roboterarms (201b), um in Reaktion auf eine Benutzerinteraktion mit der distaleren Schnittstelle (370) von dem Betrieb im nachgiebigen Modus (403) zu dem Betrieb im Kalibriermodus (404) überzugehen.

7. Steuersystem (224) nach Anspruch 5 oder 6, wobei das Steuersystem (224) ferner konfiguriert ist zum:
nach dem Ermitteln des Drehpunkts, Steuern des zweiten Roboterarms (201b), sodass er in einem Instrumenteneinstellmodus (405) arbeitet, in dem die Konfiguration des zweiten Roboterarms (201b) in Reaktion auf externe Kräfte geändert werden kann, aber so eingeschränkt ist, dass ein Schnittpunkt zwischen dem zweiten chirurgischen Instrument (206) und dem bestimmten Drehpunkt beibehalten wird.

8. Steuersystem (224) nach Anspruch 7, wobei das Steuersystem (224) ferner konfiguriert ist zum:
Steuern des zweiten Roboterarms (201b), um von dem Betrieb im Kalibriermodus (404) zu dem Betrieb im Instrumenteneinstellmodus (405) überzugehen;
Steuern des zweiten Roboterarms (201b), um von dem Betrieb im Instrumenteneinstellmodus (405) zu dem Betrieb im chirurgischen Modus (406) überzugehen; und
optional, Steuern des zweiten Roboterarms, sodass er von dem Betrieb im chirurgischen Modus (406) zu dem Betrieb im Instrumenteneinstellmodus (405) übergeht.

9. Steuersystem (224) nach einem der vorhergehenden Ansprüche, wobei jeder von dem ersten Roboterarm (201a) und dem zweiten Roboterarm (201b) ferner eine Ausrichtungsschnittstelle (350) umfasst, und wobei das Steuersystem (224) ferner konfiguriert ist zum:
Empfangen einer Eingabe, die eine gemeinsame Richtung identifiziert, in Reaktion darauf, dass ein Bediener eine Richtung unter Verwendung der Ausrichtungsschnittstelle (350) des ersten Roboterarms (201a) angibt und eine entsprechende Richtung unter Verwendung der Ausrichtungsschnittstelle (350) des zweiten Roboterarms (201b) angibt.

10. Steuersystem (224) nach Anspruch 9, wobei, in dem chirurgischen Modus (406), der zweite Roboterarm (201b) ferngesteuert wird, indem das Steuersystem (224) konfiguriert ist zum:
Empfangen von Eingaben in Bezug auf den zweiten Roboterarm (201b) an der entfernten Konsole (220);
Umwandeln der Eingaben in Steuersignale für den zweiten Roboterarm (201b) in Abhängigkeit von dem ermittelten Drehpunkt und der identifizierten gemeinsamen Richtung; und
Steuern eines oder mehrerer Gelenke (204) der Reihe von Gelenken (204) des zweiten Roboterarms (201b) in Abhängigkeit von den Steuersignalen, um die Konfiguration des zweiten Roboterarms (201b) zu steuern.

11. Steuersystem (224) nach einem der vorhergehenden Ansprüche, wobei das chirurgische Robotersystem (200) einen dritten Roboterarm (601c) umfasst, der eine Reihe von Gelenken umfasst, durch welche die Konfiguration dieses Roboterarms geändert werden kann, wobei sich die Reihe von Gelenken von einer Basis an einem proximalen Ende des Roboterarms zu einer Befestigung für ein chirurgisches Instrument an einem distalen Ende des Roboterarms erstreckt, und wobei das Steuersystem (224) ferner konfiguriert ist zum:
während des Steuerns des ersten Roboterarms (201a), sodass dieser in dem chirurgischen Modus (406) arbeitet, und bevor das zweite chirurgische Instrument (201b) in den Port (317) eingeführt werden kann, Steuern des dritten Roboterarms (201c), sodass ein drittes chirurgisches Instrument, das an dem dritten Roboterarm angebracht ist, aus dem Körper des Patienten zurückgezogen werden kann, und/oder
wobei das Steuersystem (224) ferner konfiguriert ist, zu ermöglichen, dass das dritte chirurgische Instrument aus dem Körper des Patienten zurückgezogen wird, durch:
Ermöglichen des Änderns der Konfiguration des dritten Roboterarms (601c) in Reaktion auf externe Kräfte, wobei die Bewegungsfreiheit des dritten Roboterarms (601c) derart begrenzt ist, dass sich das dritte chirurgische Instrument nur linear in Richtungen koaxial zu der Längsachse des dritten chirurgischen Instruments und weg von dem Körper des Patienten bewegen kann.

12. Steuersystem (224) nach Anspruch 11, wobei der dritte Roboterarm (601c) ferner einen oder mehrere Kraftsensoren umfasst, die konfiguriert sind, externe Kräfte an einem oder mehreren Gelenken der Reihe von Gelenken des dritten Roboterarms (601c) zu erfassen, und einen oder mehrere Motoren, die konfiguriert sind, ein oder mehrere Gelenke der Reihe von Gelenken des dritten Roboterarms (601c) anzutreiben, und wobei das Steuersystem (224) ferner konfiguriert ist zum:
Auflösen externer Kräfte, die von dem einen oder den mehreren Kraftsensoren erfasst werden, um die Komponenten der Kräfte parallel zu der Längsachse des dritten chirurgischen Instruments und weg von dem Körper des Patienten zu ermitteln; und
Steuern des einen oder der mehreren Motoren, um ein oder mehrere Gelenke der Reihe von Gelenken des dritten Roboterarms in Abhängigkeit von den Komponenten der Kräfte parallel zu der Längsachse des dritten chirurgischen Instruments anzutreiben, um die Konfiguration des dritten Roboterarms zu ändern.

13. Steuersystem (224) nach einem der vorhergehenden Ansprüche, wobei das Steuersystem (224) ferner konfiguriert ist zum: während des Steuerns des ersten Roboterarms (201a), sodass er in dem chirurgischen Modus (406) arbeitet, und bevor gestattet wird, dass das zweite chirurgische Instrument (206) in den Port (317) eingeführt wird, Steuern des zweiten Roboterarms (201b), sodass das zweite chirurgische Instrument (206) aus dem Körper des Patienten zurückgezogen werden kann; und Steuern des zweiten Roboterarms (201b), sodass das zweite chirurgische Instrument (206) nach Durchführung einer Wartungsaufgabe an dem zweiten Roboterarm (201b) in den Körper des Patienten eingeführt werden kann, und
wobei optional das Steuersystem (224) ferner konfiguriert ist, zu ermöglichen, dass das zweite chirurgische Instrument (206) aus dem Körper des Patienten zurückgezogen wird, durch:
Ermöglichen des Änderns der Konfiguration des zweiten Roboterarms (201b) in Reaktion auf externe Kräfte, wobei die Bewegungsfreiheit des zweiten Roboterarms (201b) derart begrenzt ist, dass sich das zweite chirurgische Instrument (206) nur linear in Richtungen parallel zu der Längsachse des zweiten chirurgischen Instruments (206) bewegen kann.

14. Steuersystem (224) nach Anspruch 13, wobei der zweite Roboterarm (201b) ferner einen oder mehrere Kraftsensoren (308a-h) umfasst, die konfiguriert sind, externe Kräfte an einem oder mehreren Gelenken (204) der Reihe von Gelenken (204) des zweiten Roboterarms (201b) zu erfassen, und einen oder mehrere Motoren (310a-h), die konfiguriert sind, ein oder mehrere Gelenke (204) der Reihe von Gelenken (204) des zweiten Roboterarms (201b) anzutreiben, und wobei das Steuersystem (224) ferner konfiguriert ist zum:
Auflösen externer Kräfte, die von dem einen oder den mehreren Kraftsensoren (308a-h) erfasst werden, um die Komponenten der Kräfte parallel zu der Längsachse des zweiten chirurgischen Instruments (206) zu ermitteln; und
Steuern des einen oder der mehreren Motoren (310a-h), um ein oder mehrere Gelenke der Reihe von Gelenken des zweiten Roboterarms (201b) in Abhängigkeit von den Komponenten der Kräfte parallel zu der Längsachse des zweiten chirurgischen Instruments (206) anzutreiben, um die Konfiguration des zweiten Roboterarms (201b) zu ändern.

## Revendications

1. Système de commande (224) d'un système robotique chirurgical (200), le système robotique chirurgical (200) comprenant un premier bras de robot (201a) et un deuxième bras de robot (201b), chacun des premier et deuxième bras de robot (201a, 201b) comprenant une série d'articulations (204) par lesquelles la configuration de ce bras de robot (201a, 201b) peut être modifiée, la série d'articulations (204) s'étendant depuis une base (209) au niveau d'une extrémité proximale du bras de robot (201a, 201b) jusqu'à une fixation pour un instrument chirurgical (206) au niveau d'une extrémité distale du bras de robot (201a, 201b), le système de commande (224) étant configuré pour reconfigurer le système robotique chirurgical (200) par :
la commande du premier bras de robot pour qu'il fonctionne dans un mode chirurgical (406) dans lequel un premier instrument chirurgical (206) fixé à ce premier bras de robot (201a) est à l'intérieur du corps d'un patient et la configuration du premier bras de robot et du premier instrument chirurgical est commandée en réponse à des entrées reçues au niveau d'une console de chirurgien à distance ; et
**caractérisé en ce que** le système de commande (224) est configuré pour reconfigurer le système robotique chirurgical (200) par :
tandis que le premier bras de robot (201a) fonctionne dans le mode chirurgical (406) :
(i) la commande du deuxième bras de robot (201b) de manière à permettre à un deuxième instrument chirurgical (206) fixé au deuxième bras de robot (201b) d'être inséré dans un orifice (317) dans le corps du patient ;
(ii) la détermination d'un point d'appui autour duquel pivote le deuxième instrument chirurgical (206) lorsque la configuration du deuxième bras de robot (201b) est modifiée tandis que le deuxième instrument chirurgical (206) est à l'intérieur de l'orifice (317) ; et
(iii) la commande du deuxième bras de robot (201b) pour qu'il fonctionne dans le mode chirurgical (406) dans lequel le deuxième instrument chirurgical (206) fixé à ce deuxième bras de robot (201b) est à l'intérieur du corps du patient et la configuration du deuxième bras de robot (201b) et du deuxième instrument chirurgical (206) est commandée en réponse à des entrées reçues au niveau de la console de chirurgien à distance (220) tout en maintenant une intersection entre le deuxième instrument chirurgical (201b) et le point d'appui déterminé.

2. Système de commande (224) selon la revendication 1, dans lequel le point d'appui est déterminé par :
la commande du deuxième bras de robot (201b) de manière à permettre que sa configuration soit modifiée en réponse à des forces externes tandis que le deuxième instrument chirurgical (206) est à l'intérieur de l'orifice (317) ; et
la détermination du point d'appui, le point d'appui étant le point autour duquel l'instrument chirurgical (206) du deuxième bras de robot (201b) pivote tout en étant à l'intérieur de l'orifice (317).

3. Système de commande (224) selon la revendication 2, dans lequel le deuxième bras de robot (201b) comprend en outre un ou plusieurs capteurs de force (308a-h) configurés pour capter des forces externes au niveau d'une ou plusieurs articulations (204) de la série d'articulations (204) du deuxième bras de robot (201b), et un ou plusieurs moteurs (310a-h) configurés pour entraîner une ou plusieurs articulations (204) de la série d'articulations du deuxième bras de robot (201b), et le système de commande (224) est en outre configuré pour :
commander les un ou plusieurs moteurs (310a-h) de manière à entraîner une ou plusieurs articulations (204) de la série d'articulations (204) du deuxième bras de robot (201b) en fonction des forces externes captées par les un ou plusieurs capteurs de force (308a-h) de manière à modifier la configuration du deuxième bras de robot (201b).

4. Système de commande (224) selon une quelconque revendication précédente, dans lequel le deuxième bras de robot (201b) comprend en outre un ou plusieurs capteurs de position (307a-h) configurés pour capter la position d'une ou plusieurs articulations (204) de la série d'articulations (204) du deuxième bras de robot (201b) et pour enregistrer la position d'une ou plusieurs articulations (204) de la série d'articulations (204) du deuxième bras de robot (201b) à une pluralité d'occasions alors que la configuration du deuxième bras de robot (201b) est en cours de modification, et le système de commande (224) est en outre configuré pour :
déterminer, pour chaque occasion, une position de l'extrémité distale du deuxième bras de robot (201b) en fonction des une ou plusieurs positions d'articulation enregistrées respectives ;
déterminer, pour chaque occasion, un vecteur du deuxième instrument chirurgical (201b) à partir de la position déterminée de l'extrémité distale du deuxième bras de robot (201b) en fonction des une ou plusieurs positions d'articulation enregistrées respectives ; et
déterminer le point d'intersection des vecteurs déterminés du deuxième instrument chirurgical (201b) de manière à déterminer le point d'appui.

5. Système de commande (224) selon une quelconque revendication précédente, le système de commande (224) étant en outre configuré pour :
déterminer le point d'appui lors de la commande du deuxième bras de robot (201b) pour qu'il fonctionne dans un mode d'étalonnage (404) ; et
commander le deuxième bras de robot (201b) pour passer du fonctionnement en mode d'étalonnage (404) au fonctionnement en mode chirurgical (406), et
facultativement dans lequel le deuxième bras de robot (201b) comprend en outre une interface plus distale et une interface moins distale, et le système de commande (224) est en outre configuré pour :
commander le deuxième bras de robot (201b) pour passer du fonctionnement en mode d'étalonnage (404) au fonctionnement en mode chirurgical (406) en réponse à une interaction de l'opérateur avec l'interface plus distale (370).

6. Système de commande (224) selon la revendication 5, le système de commande (224) étant en outre configuré pour :
commander le deuxième bras de robot (201b) de manière à permettre au deuxième instrument chirurgical (206) d'être inséré dans l'orifice (317) en commandant le deuxième bras de robot (201b) pour qu'il fonctionne dans un mode souple (403) dans lequel la configuration du deuxième bras de robot (201b) peut être modifiée en réponse à des forces externes ; et
commander le deuxième bras de robot (201b) pour passer du fonctionnement en mode souple (403) au fonctionnement en mode d'étalonnage (404), et
facultativement le système de commande étant en outre configuré pour :
commander le deuxième bras de robot (201b) pour passer du fonctionnement en mode souple (403) au fonctionnement en mode d'étalonnage (404) en réponse à une interaction de l'utilisateur avec l'interface plus distale (370).

7. Système de commande (224) selon la revendication 5 ou 6, le système de commande (224) étant en outre configuré pour :
après la détermination du point d'appui, commander le deuxième bras de robot (201b) pour qu'il fonctionne dans un mode de réglage d'instrument (405) dans lequel la configuration du deuxième bras de robot (201b) peut être modifiée en réponse à des forces externes mais est contrainte de sorte qu'une intersection est maintenue entre le deuxième instrument chirurgical (206) et le point d'appui déterminé.

8. Système de commande (224) selon la revendication 7, le système de commande (224) étant en outre configuré pour :
commander le deuxième bras de robot (201b) pour passer du fonctionnement en mode d'étalonnage (404) au fonctionnement en mode de réglage d'instrument (405) ;
commander le deuxième bras de robot (201b) pour passer du fonctionnement en mode de réglage d'instrument (405) au fonctionnement en mode chirurgical (406) ; et
facultativement, commander le deuxième bras de robot pour passer du fonctionnement en mode chirurgical (406) au fonctionnement en mode de réglage d'instrument (405).

9. Système de commande (224) selon une quelconque revendication précédente, dans lequel chacun du premier bras de robot (201a) et du deuxième bras de robot (201b) comprend en outre une interface d'orientation (350), et le système de commande (224) est en outre configuré pour :
recevoir une entrée identifiant une direction commune en réponse à un opérateur indiquant une direction en utilisant l'interface d'orientation (350) du premier bras de robot (201a) et indiquant une direction correspondante en utilisant l'interface d'orientation (350) du deuxième bras de robot (201b).

10. Système de commande (224) selon la revendication 9, dans lequel, dans le mode chirurgical (406), le deuxième bras de robot (201b) est commandé à distance par le système de commande (224) qui est configuré pour :
recevoir, à la console distante (220), des entrées relatives au deuxième bras de robot (201b) ;
convertir les entrées en signaux de commande pour le deuxième bras de robot (201b) en fonction du point d'appui déterminé et de la direction commune identifiée ; et
commander une ou plusieurs articulations (204) de la série d'articulations (204) du deuxième bras de robot (201b) en fonction des signaux de commande de manière à commander la configuration du deuxième bras de robot (201b).

11. Système de commande (224) selon une quelconque revendication précédente, dans lequel le système robotique chirurgical (200) comprend un troisième bras de robot (601c) comprenant une série d'articulations par lesquelles la configuration de ce bras de robot peut être modifiée, la série d'articulations s'étendant depuis une base au niveau d'une extrémité proximale du bras de robot jusqu'à une fixation pour un instrument chirurgical au niveau d'une extrémité distale du bras de robot, et le système de commande (224) est en outre configuré pour :
tout en commandant le premier bras de robot (201a) pour qu'il fonctionne dans le mode chirurgical (406) et avant de permettre au deuxième instrument chirurgical (201b) d'être inséré dans l'orifice (317), commander le troisième bras de robot (201c) de manière à permettre à un troisième instrument chirurgical fixé au troisième bras de robot d'être rétracté du corps du patient, et/ou le système de commande (224) étant en outre configuré pour permettre au troisième instrument chirurgical d'être rétracté du corps du patient par :
la possibilité de modifier la configuration du troisième bras de robot (601c) en réponse à des forces externes, la liberté de mouvement du troisième bras de robot (601c) étant limitée de telle sorte que le troisième instrument chirurgical ne peut se déplacer linéairement que dans des directions coaxiales à l'axe longitudinal du troisième instrument chirurgical et en s'éloignant du corps du patient.

12. Système de commande (224) selon la revendication 11, dans lequel le troisième bras de robot (601c) comprend en outre un ou plusieurs capteurs de force configurés pour capter des forces externes au niveau d'une ou plusieurs articulations de la série d'articulations du troisième bras de robot (601c), et un ou plusieurs moteurs configurés pour entraîner une ou plusieurs articulations de la série d'articulations du troisième bras de robot (601c), et le système de commande (224) est en outre configuré pour :
résoudre les forces externes captées par les un ou plusieurs capteurs de force de manière à déterminer les composantes des forces parallèles à l'axe longitudinal du troisième instrument chirurgical et s'éloignant du corps du patient ; et
commander les un ou plusieurs moteurs de manière à entraîner une ou plusieurs articulations de la série d'articulations du troisième bras de robot en fonction des composantes des forces parallèles à l'axe longitudinal du troisième instrument chirurgical de manière à modifier la configuration du troisième bras de robot.

13. Système de commande (224) selon une quelconque revendication précédente, dans lequel le système de commande (224) est en outre configuré pour ; tout en commandant le premier bras de robot (201a) pour qu'il fonctionne en mode chirurgical (406) et avant de permettre au deuxième instrument chirurgical (206) d'être inséré dans le port (317), commander le deuxième bras de robot (201b) de manière à permettre au deuxième instrument chirurgical (206) d'être rétracté du corps du patient ; et commander le deuxième bras de robot (201b) de manière à permettre au deuxième instrument chirurgical (206) d'être inséré dans le corps du patient après qu'une tâche de maintenance a été réalisée sur le deuxième bras de robot (201b), et
facultativement le système de commande (224) étant en outre configuré pour permettre au deuxième instrument chirurgical (206) d'être rétracté du corps du patient par :
la possibilité de modifier la configuration du deuxième bras de robot (201b) en réponse à des forces externes, la liberté de mouvement du deuxième bras de robot (201b) étant limitée de telle sorte que le deuxième instrument chirurgical (206) ne peut se déplacer linéairement que dans des directions parallèles à l'axe longitudinal du deuxième instrument chirurgical (206).

14. Système de commande (224) selon la revendication 13, dans lequel le deuxième bras de robot (201b) comprend en outre un ou plusieurs capteurs de force (308a-h) configurés pour capter des forces externes au niveau d'une ou plusieurs articulations (204) de la série d'articulations (204) du deuxième bras de robot (201b), et un ou plusieurs moteurs (310a-h) configurés pour entraîner une ou plusieurs articulations (204) de la série d'articulations (204) du deuxième bras de robot (201b), et le système de commande (224) est en outre configuré pour :
résoudre les forces externes captées par les un ou plusieurs capteurs de force (308a-h) de manière à déterminer les composantes des forces parallèles à l'axe longitudinal du deuxième instrument chirurgical (206) ; et
commander les un ou plusieurs moteurs (310a-h) de manière à entraîner une ou plusieurs articulations de la série d'articulations du deuxième bras de robot (201b) en fonction des composantes des forces parallèles à l'axe longitudinal du deuxième instrument chirurgical (206) de manière à modifier la configuration du deuxième bras de robot (201b).
